# EUROPEAN PATENT APPLICATION

(11) **EP 4 480 406 A1**
(43) Date of publication of application: **25.12.2024**
(21) Application number: 24177728.3
(22) Date of filing: 23.05.2024
(51) Int. Cl.: A61B 5/107

(54) **FOOT PRONATION ESTIMATION APPARATUS, METHOD FOR ESTIMATING FOOT PRONATION, AND FOOT PRONATION ESTIMATION PROGRAM**

(30) Priority: 23.06.2023 JP 2023103229
(71) Applicant: ASICS Corporation, Kobe-shi, Hyogo 650-8555 (JP)
(72) Inventor: Nakayama, Kazunaga, Hyogo, 650-8555 (JP); Nakaya, Mai, Hyogo, 650-8555 (JP); Tsutsui, Masayuki, Hyogo, 650-8555 (JP)
(74) Representative: Marks & Clerk LLP

(57) **Abstract**

Provided is a foot pronation estimation apparatus (50), in which a pronation estimator (72) estimates a degree of pronation of a foot, with reference to comparison between a feature value of a predetermined part and a reference value. A feature value extractor (60) extracts, from a top-view image, feature values of a plurality of parts including at least a width of foot and an amount of a recess of an arch shape. A pronation estimator (72) estimates a degree of pronation of the foot, with reference to comparison of each of the feature values of the plurality of parts including at least the foot width and the amount of the recess of the arch shape, with an individually corresponded reference value. The feature values of the plurality of parts further include foot length and an amount of protrusion in an outline shape near a little toe ball.

## Description

### BACKGROUND

### 1. Technical Field

The present disclosure relates to a foot pronation estimation apparatus.

### 2. Background Information

Some running shoe products are occasionally categorized by types of pronation (pronation type). The categorization is often made into for runners with overpronation, and for other runners (with neutral pronation, and underpronation). The runners can reduce risk of failure while maintaining appropriate pronation, with use of running shoes suitable for their own pronation types.

Now, there is a known technique of estimating an arch height ratio or foot flexibility of a customer, by measuring a footprint or a foot pressure distribution, thereby selecting a shoe type well fit to the customer (see, WO2005/006905A1, for example).

### SUMMARY

Selecting the running shoes according to the pronation type is on the premise that the person can recognize the pronation type of its own. Determination of the pronation type has, however, usually needed to shoot running posture on a treadmill from the back to analyze steps, inevitably needing a facility having equipment for such shooting and analysis. On the other hand, although the technique of determination disclosed in WO2005/006905A1 can estimate the pronation type from the footprint or the foot pressure distribution, this needs use of a measuring instrument such as optical sensor or pressure sensor, making it not always easy for the ordinary people to make decision.

Meanwhile for the shoe manufacturers who faithfully categorize their running shoes by the pronation type, it is desirable to avoid any mismatch such that a person without overpronation improperly wears the shoes for the people with overpronation. In particular, in recent years in which also online shoe sales have become common, desired is establishment of a technique capable of easily determining the pronation type, even in an environment without dedicated equipment.

The present disclosure has been made in view of these circumstances, wherein an object of which is to provide a novel technique for easily determining the pronation type.

Aimed at solving the issue, a foot pronation estimation apparatus of one embodiment includes: an image acquirer structured to acquire at least a top-view image of a foot of a measured person, as an image that depicts a contour shape in a top view of the foot; a feature value extractor structured to extract, from the top-view image, a feature value of a predetermined part that depicts a top-view contour shape of the foot; and a pronation estimator structured to estimate a degree of pronation of the foot, with reference to comparison between the feature value of the predetermined part and a reference value.

Another aspect of the present disclosure relates to a method for estimating foot pronation. The method includes: acquiring at least a top-view image of a foot of a measured person, as an image that depicts a contour shape in a top view of the foot; extracting, from the top-view image by image processing, a feature value of a predetermined part that depicts a top-view contour shape of the foot; and estimating a degree of pronation of the foot, with reference to a comparison process between the feature value of the predetermined part and a reference value.

### BRIEF DESCRIPTION OF THE DRAWINGS

Fig. 1 is a drawing illustrating a basic structure of a foot pronation estimation system;
Fig. 2 is a functional block diagram illustrating structures of the individual units in a foot pronation estimation apparatus;
Fig. 3 is a drawing illustrating a method for measuring a foot length and foot width;
Fig. 4 is a drawing illustrating a method for measuring the amount of medial recess and lateral protrusion;
Fig. 5 is a chart summarizing relations between pronation types and shoe types;
Fig. 6 is a chart summarizing results of collation between the subject persons classified by the decision tree analysis, and the pronation types classified by the three-dimensional valgus angle;
Fig. 7 is a tree diagram schematically illustrating results of a decision tree analysis of foot pronation estimation based on three factors including foot length, foot width, and the amount of medial recess;
Fig. 8 is a flow chart illustrating a flow of a foot pronation estimation process with use of a foot pronation estimation algorithm based on three factors including foot length, foot width, and the amount of medial recess;
Fig. 9 is a tree diagram schematically illustrating results of a decision tree analysis of foot pronation estimation based on three factors including foot width, the amount of medial recess, and the amount of lateral protrusion; and
Fig. 10 is a flow chart illustrating a flow of the foot pronation estimation process with use of a foot pronation estimation algorithm based on three factors including foot width, the amount of medial recess, and the amount of lateral protrusion.

### DETAILED DESCRIPTION

The disclosure will now be described by reference to the preferred embodiments. This does not intend to limit the scope of the present invention, but to exemplify the invention.

The present disclosure will be explained below on the basis of preferred embodiments, referring to the attached drawings. All similar or equivalent constituents, members and processes illustrated in the individual drawings will be given same reference numerals, so as to properly avoid redundant explanations.

In this embodiment, a feature value that depicts a top-view contour shape of a foot of a measured person in a sitting posture is extracted, from a top-view image of the foot, and a degree of pronation of the foot is estimated by comparison with a reference value. The top-view image is an image that depicts a contour shape of the foot of the measured person in a top view. Since the top-view image contains a contour shape of the entire foot, including toe shape and heel shape, so that the feature value of a foot part including the foot length and the foot width may be extracted from the top-view image. A technique of estimating the pronation type from the feature value of the food part employed herein is such that relations between the feature values of the foot parts, measured for a plurality of subjects, and the pronation types are preliminarily analyzed with use of the decision tree, and the pronation type is estimated from the analytical results with use of an estimation algorithm.

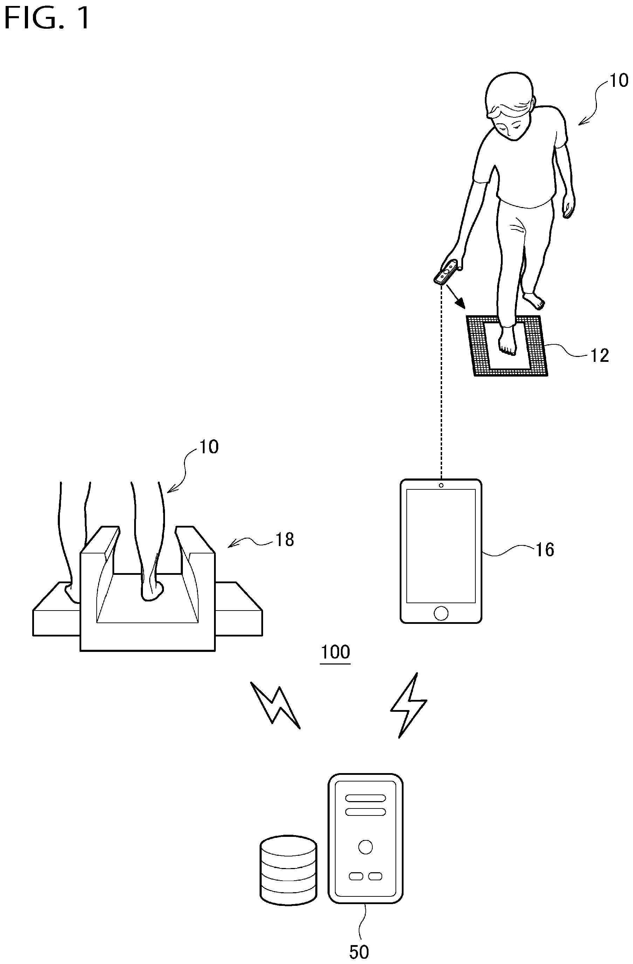

Fig. 1 is a drawing illustrating a basic structure of a foot pronation estimation system 100. The foot pronation estimation system 100 includes a device which acquires a top-view image of the foot, such as an information terminal 16 or a three-dimensional foot shape measuring device 18, and a foot pronation estimation apparatus 50. Assuming now an exemplary case where a measured person 10, who is a runner considering purchase of running shoes categorized by the pronation type, uses the information terminal 16 or the three-dimensional foot shape measuring device 18, in order to determine the pronation type of its foot. The measured person 10 acquires a top view image for each foot, and determines the pronation type for each of the left and right feet.

The information terminal 16 may be a personal digital assistance such as smartphone or tablet terminal, or may be a personal computer. The information terminal 16 is typically constituted by a combination of hardware such as camera module, range sensor, microprocessor, touch panel, memory, and communication module. The information terminal 16 may be a general-purpose information terminal such as a smartphone or a tablet which is owned by the measured person 10 or provided by a shoe shop. The information terminal 16 displays, on a screen thereof, information which is accessible through a web browser by visiting a website presented by the foot pronation estimation apparatus 50, or which is provided from the foot pronation estimation apparatus 50 through application software run on the information terminal 16.

When using the information terminal 16, the measured person 10 in a sitting posture shoots and captures an image of the foot, with a built-in camera of the information terminal 16 such as a mobile phone terminal of its own. With an exemplary technique by which the entire foot is shot directly from above, it would be difficult to cover a contour shape of the entire foot with a single shot of image, since the ankle is inevitably captured in the image despite every effort of making the heel fully recognizable. The shooting may, therefore, be on the premise of obtaining an image in which at least the outlines of the toe, the heel, and the left and right sides of the front to middle parts of the foot are captured.

The information terminal 16, if equipped with a three-dimensional scanner function typically based on the light detection and ranging (LiDAR) technology, may scan the periphery of the foot to produce a three-dimensional model of the foot shape, and then may acquire the top-view image, which is a two-dimensional image, from the three-dimensional model. The information terminal 16, even if not equipped with the three-dimensional scanner function typically based on LiDAR, may produce the three-dimensional model of the foot shape by using image synthesis such as photogrammetry, and then may acquire the two-dimensional image, which is the top-view image, from the three-dimensional model. The measured person 10 may place its foot on a dedicated measurement mat 12, may scan its own foot shape with use of a camera function and a foot shape acquisition application of the information terminal 16, and may produce the three-dimensional model of the foot shape.

When using the three-dimensional foot shape measuring device 18, the measured person 10 may generate a three-dimensional model of the foot, typically with use of the three-dimensional foot shape measuring device 18 installed at a shoe shop, and may acquire the top view image, which is a two-dimensional image, from the three-dimensional model. The three-dimensional foot shape measuring device 18 acquires three-dimensional data of the foot shape by laser measurement. A measurement value of the foot shape scanned with the three-dimensional foot shape measuring device 18, as a result of three-dimensional measurement, may be transmitted from the three-dimensional foot shape measuring device 18 to the foot pronation estimation apparatus 50, and the top view image, which is a two-dimensional image, may be produced from the three-dimensional model in the foot pronation estimation apparatus 50.

In a modified example, a side-view image of the foot of the measured person 10 may be acquired, in place of the top-view image. Still alternatively, both of the top-view image and the side-view image of the foot of the measured person 10 may be acquired. Yet alternatively, the top-view image or the side-view image of the foot of the measured person 10 in a standing posture may be acquired.

The foot pronation estimation apparatus 50 is connected to the plurality of information terminals 16 or the three-dimensional foot shape measuring devices 18, through a network line such as the Internet or a local area network (LAN), or through a communication means such as wireless communication. The foot pronation estimation apparatus 50 may alternatively be implemented as a server that estimates the pronation type of the foot, with reference to the measurement data transmitted from the plurality of information terminals 16 or the three-dimensional foot shape measuring devices 18. The foot pronation estimation apparatus 50 may be constituted by a single server computer, or by a combination of a plurality of server computers. Note that the "foot pronation estimation apparatus" stated in the claims may refer to the entire foot pronation estimation system 100, or may refer to the foot pronation estimation apparatus 50. In this embodiment, the foot pronation estimation apparatus 50 substantially corresponds to the "foot pronation estimation apparatus" stated in claims, since many of characteristic functions of the "foot pronation estimation apparatus" stated in claims are implemented as those owned by the foot pronation estimation apparatus 50. However in the modified example, the "foot pronation estimation apparatus" stated in claims may be implemented by cooperation of the foot pronation estimation apparatus 50 and the information terminal 16, in which processing such as acquisition of the top-view image, extraction of the feature value of the foot part, and output of estimated result of the foot pronation type are implemented by the information terminal 16.

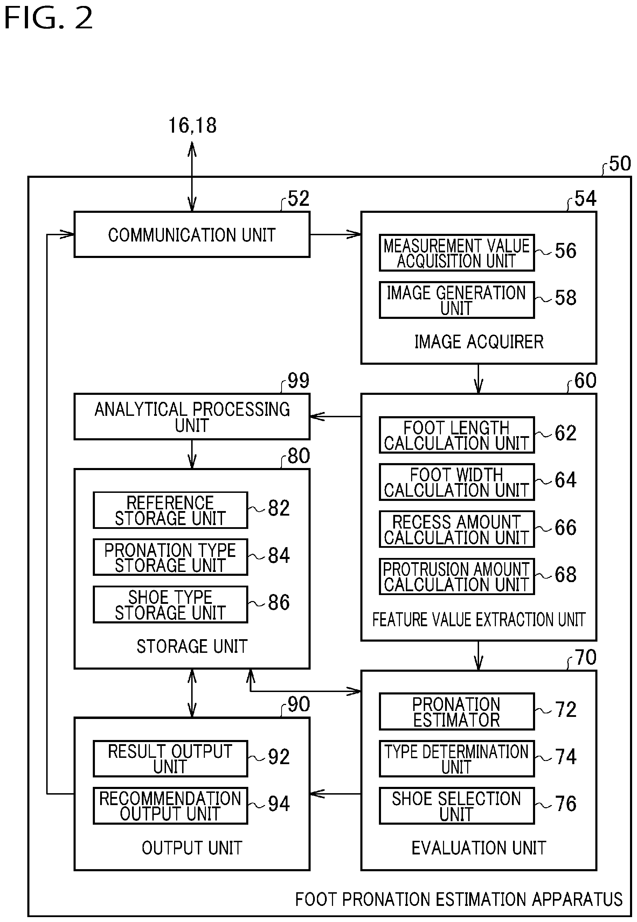

Fig. 2 is a functional block diagram illustrating structures of the individual units in the foot pronation estimation apparatus 50. Fig. 2 illustrates functional blocks of the foot pronation estimation apparatus 50, which can be implemented by cooperation of various hardware structures and software structures. Those skilled in the art will easily understand that these functional blocks may be implemented in various ways, such as solely by hardware, solely by software, or by combination of them. The foot pronation estimation apparatus 50 is typically constituted by a combination of hardware such as microprocessor, memory, display, and communication module. The foot pronation estimation apparatus 50 may be a server computer which is constructed and managed by a shoe manufacturer. Now, a program having functions below runs on the foot pronation estimation apparatus 50. The foot pronation estimation apparatus 50 is functionally constituted by a communication unit 52, an image acquirer 54, a feature value extractor 60, an evaluation unit 70, a storage 80, an output unit 90, and an analytical processing unit 99. The communication unit 52 of the foot pronation estimation apparatus 50 and the information terminal 16 or the three-dimensional foot shape measuring device 18 are connected through a network.

The image acquirer 54 acquires the top-view image, transmitted from the information terminal 16 or from the three-dimensional foot shape measuring device 18, through the communication unit 52. The image acquirer 54 acquires, as the top-view image, an image that depicts the top-view contour shape of the foot of the measured person 10 in a sitting posture. The image acquirer 54 acquires the top-view image for each of the left and right feet. Acquisition of the images may, however, be directed to either the left or right foot, rather than being directed to both feet. In a modified example, the image acquirer 54 may acquire the top-view image and the side-view image of the foot of the measured person 10. In a modified example, the image acquirer 54 may alternatively acquire the top-view image or the side-view image of the foot of the measured person 10 in a standing posture.

The image acquirer 54 includes a measurement value acquisition unit 56 and an image generation unit 58. In a case where the information terminal 16 or the three-dimensional foot shape measuring device 18 transmits the three-dimensional measurement value of the foot shape rather than the top-view image, the measurement value acquisition unit 56 acquires the three-dimensional measurement value through the communication unit 52. In this case, the image generation unit 58 generates a three-dimensional model from the three-dimensional measurement value, and generates the top-view image, which is the two-dimensional image, from the three-dimensional model. A technique for generating a three-dimensional model from a three-dimensional measured value will not be explained here, since it has been already known to the public.

The feature value extractor 60 extracts the feature values of a plurality of foot parts from the top-view image. The feature values of the plurality of foot parts include at least one of (1) foot length, (2) foot width, (3) the amount of recess of arch shape (referred to as "the amount of medial recess", hereinafter), and (4) the amount of protrusion in the outline shape near the fifth metatarsal head (referred to as "the amount of lateral protrusion", hereinafter). The feature value extractor 60 includes a foot length calculation unit 62, a foot width calculation unit 64, a recess amount calculation unit 66, and a protrusion amount calculation unit 68. The foot length calculation unit 62 calculates the foot length from the top-view image. The foot width calculation unit 64 calculates the foot width from the top-view image. The recess amount calculation unit 66 calculates the medial recess from the top-view image. The protrusion amount calculation unit 68 calculates the lateral protrusion from the top-view image.

A pronation estimator 72 estimates the degree of pronation of the foot, with reference to comparison of the feature value including at least one of the foot length, foot width, the amount of medial recess, and the amount of lateral protrusion, with the reference value. The pronation estimator 72 estimates which one of a plurality of pronation types applies to the foot of the measured person 10, with use of a foot pronation estimation algorithm having been preliminarily determined in the design phase by the decision tree analysis described later. The pronation estimator 72 estimates the pronation type for each of the left and right feet of the measured person 10.

The storage 80 includes a reference storage 82, a pronation type storage 84, and a shoe type storage 86. The reference storage 82 stores the respective reference values related to the feature values of the plurality of foot parts determined by decision tree analysis described later.

The pronation type storage 84 stores a plurality of pronation types classified by the degree of pronation of the foot, including at least a first type whose degree of pronation is larger than a predetermined standard and a second type whose degree of pronation is equal to or smaller than the predetermined standard. The predetermined standard as used herein is, for example, "neutral pronation" in which the heel tilts moderately to the medial side at the time of landing.

The shoe type storage 86 stores shoe product model information according to the shoe types classified by the mode of dealing with pronation of foot, including a first shoe type adapted to a first type in which a degree of pronation is larger than a predetermined standard, and a second shoe type adapted to a second type in which a degree of pronation is equal to or smaller than the predetermined standard.

The evaluation unit 70 includes the pronation estimator 72, a type determiner 74, and a shoe selection unit 76. The pronation estimator 72 estimates the pronation type of the foot of the measured person 10, with use of a foot pronation estimation algorithm described later. The type determiner 74 determines the shoe type recommendable to the measured person 10 from among the plurality of shoe types, with reference to the degree of pronation estimated by the pronation estimator 72. The shoe selection unit 76 selects a shoe product model that corresponds to the shoe type determined by the type determiner 74, from the shoe type storage 86.

The output unit 90 includes a result output unit 92, and a recommendation output unit 94. The result output unit 92 transmits the information regarding the pronation type of the foot of the measured person 10 determined by the pronation estimator 72, and information regarding the shoe type suitable for the foot of the measured person 10 determined by the type determiner 74, to the information terminal 16 through the communication unit 52. The recommendation output unit 94 transmits information regarding the shoe product model selected by the shoe selection unit 76 and recommendable to the measured person 10, to the information terminal 16 through the communication unit 52.

The analytical processing unit 99 determines, in a design phase of the foot pronation estimation apparatus 50, a foot pronation estimation algorithm for estimating whether the degree of pronation of the foot applies to the first type or the second type, according to a plurality of feature values measured from images of the feet of a plurality of subjects. The analytical processing unit 99 determines a foot pronation estimation algorithm by the decision tree analysis, particularly by the regression tree analysis. The decision tree analysis is preliminarily implemented before the foot pronation is estimated for the measured person 10, which is typically in the design phase of the foot pronation estimation system 100. Determination procedure and determination threshold for each feature value determined by the decision tree analysis are employed as a foot pronation estimation algorithm, in the foot pronation estimation processing by the pronation estimator 72. The determination threshold for each feature value determined by the decision tree analysis is stored in the reference storage 82, as a determination reference value for each feature value in the foot pronation estimation process. Candidates of the feature value of each foot part used for estimating the foot pronation include foot length, foot width, the amount of medial recess, and the amount of lateral protrusion, all of which are extractable from the top-view image.

The decision tree analysis in the design phase is implemented according to the procedures below. First, a subject running barefoot is shot with a motion capture system, a three-dimensional valgus angle of the heel is detected from the video, and an actual pronation type is determined. In particular, the pronation type is determined to be overpronation or the first type, if the valgus angle of the heel is found to exceed a predetermined threshold value X. Note that another method applicable herein may be such as shooting the subject running barefoot on a treadmill from the rear, and a two-dimensional valgus angle of the heel is detected from the captured video. Also note that the subject may run in shoes which are not pronation-suppressive, rather than running barefoot. Such pronation type determination is implemented on n subjects, which are then classified by the pronation types. On the other hand, feature values of a plurality of foot parts in these subjects are measured, as target data for the decision tree analysis. For example, foot length, foot width, the amount of medial recess, and the amount of lateral protrusion are extracted as the feature values of the foot parts, from the top-view image of the foot.

The decision tree analysis is then implemented while employing classification of n subjects depending on whether they apply to overpronation or not as an objective variable; and two or more factors from among foot length, foot width, the amount of medial recess, and the amount of lateral protrusion as explanatory variables. The decision tree analysis is implemented on all possible combination patterns of the four factors including foot length, foot width, the amount of medial recess, and the amount of lateral protrusion, to be employed as the explanatory variables, while changing the combinations among two factors, three factors, or four factors. In the decision tree analysis, the maximum depth of node is set to 5, and the element of the explanatory variable that minimizes the square error, which represents a loss function for each branched node, and a threshold thereof are determined in a regression manner.

Results of classification of the subjects determined by the decision tree analysis are then collated with results of classification of pronation type based on the three-dimensional valgus angle. A combination of the feature values, under which results of classification of all subjects were found by the collation to completely coincide, is considered to be an effective explanatory variable with which the foot pronation is highly accurately estimated. A branching condition and a branching order in the decision tree analysis, under which the foot pronation was successfully estimated with high accuracy, are employed for the foot pronation estimation algorithm used in the pronation estimator 72. The threshold determined as the branching condition for each feature value is stored in the reference storage 82, as a reference value for each feature value in the foot pronation estimation algorithm. Not only the reference value for each feature value, but also the foot pronation estimation algorithm *per se* may be stored in the reference storage 82. For example, the information stored in the reference storage 82 may be rewritten, for example, if some post-experiment based on the decision tree analysis successfully determined an algorithm capable of more accurately determining the foot pronation type, or the reference value for each feature value. This enables updating of the foot pronation estimation algorithm or the reference value for each feature value, thereby easily improving the estimation accuracy.

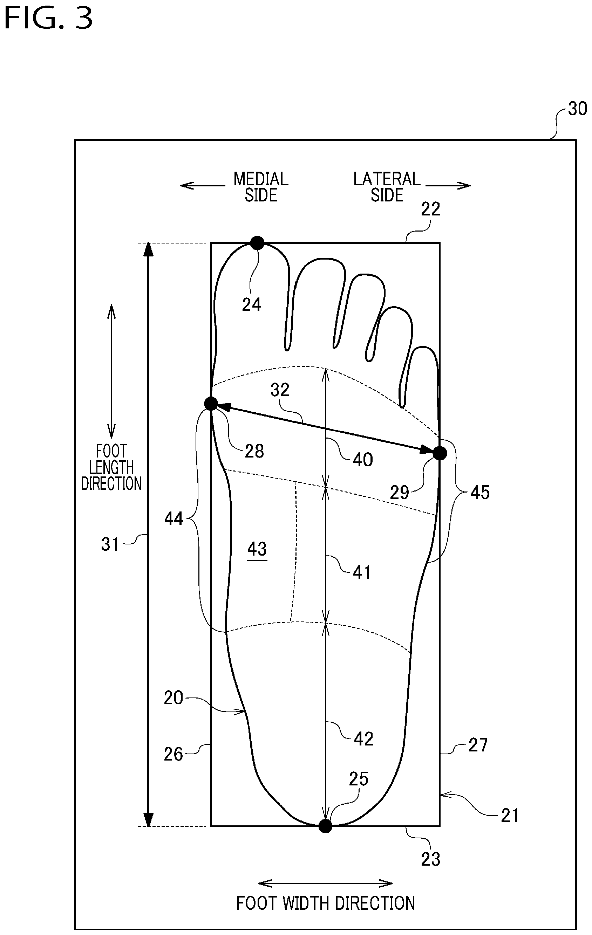

Fig. 3 illustrates a method for measuring the foot length and foot width. The feature value extractor 60 sets a bounding box 21 that surrounds a contour of a foot part 20 in a top-view image 30. The bounding box 21 is a rectangular region set as a minimum rectangle that circumscribes an overall contour of the foot part 20. Contact points of the foot part 20 with the bounding box 21 include a toe end point 24, a heel end point 25, a medial end point 28, and a lateral end point 29.

The sole part excluding the toes is mainly divided into a front foot part 40, a middle foot part 41, and a rear foot part 42. A region of the middle foot part 41, particularly on the medial side thereof, corresponds to an arch part 43. An arch shape 44 corresponds to a contour shape on the medial side (also referred to as inner foot side) that ranges from the front foot part 40 to the middle foot part 41, and mainly refers to a contour shape on the medial side of the arch part 43. The arch shape 44 is usually a shape of a part having a recess. An outline shape 45 near the little toe ball corresponds to a contour shape on the lateral side (also referred to as outer foot side) that ranges mainly from the front foot part 40 to the middle foot part 41, and mainly refers to a contour shape on the lateral side of the front foot part 40. The outline shape 45 near the little toe ball is usually a shape of a part having a protrusion.

The foot length calculation unit 62 calculates a foot length 31 in the bounding box 21, which corresponds to the distance between an upper side 22 passing through the toe end point 24 and a lower side 23 passing through the heel end point 25, that is, the length of a left side 26 passing through the medial end point 28. The medial end point 28 falls on a point given by a predetermined percentage of the left side 26, which typically accounts for 72% of the foot length 31, or 28% away from the upper side 22. The lateral end point 29 falls on a point given by a predetermined percentage of a right side 27, which typically accounts for 63% of the foot length 31, or 37% away from the upper side 22. The foot width calculation unit 64 calculates a length of a straight line connecting the medial end point 28 and the lateral end point 29 as a foot width 32.

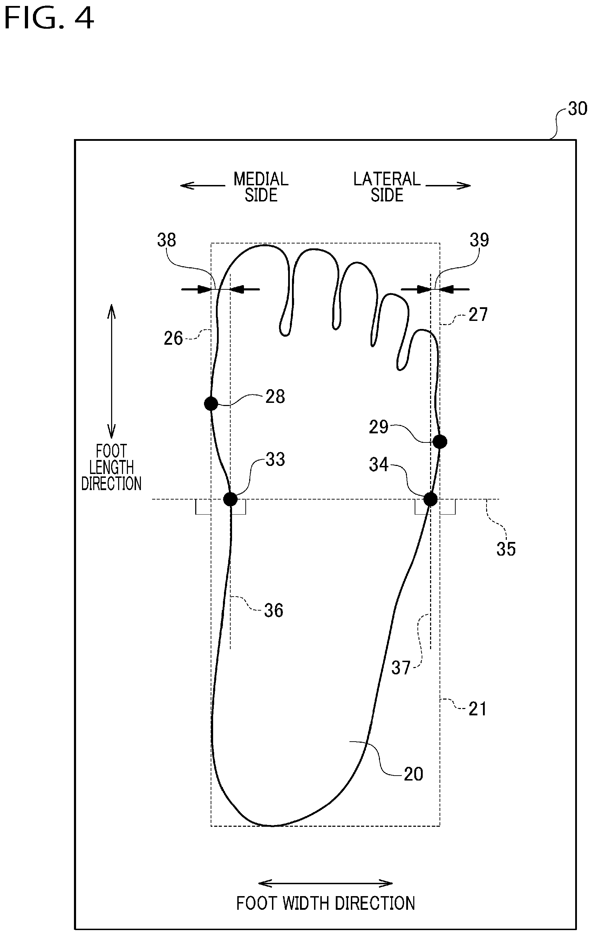

Fig. 4 illustrates a method for measuring the amount of medial recess and lateral protrusion. An amount of medial recess 38 calculated by the recess amount calculation unit 66 corresponds to the distance between an imaginary line passing through the most protruded point and an imaginary line passing through the most recessed point in the arch shape 44. That is, the amount of medial recess 38 represents a distance between the left side 26 passing through the medial end point 28 and a medial side reference line 36 passing through a deepest point 33 which is the most recessed point in the arch shape 44. The medial side reference line 36 is an imaginary line parallel to the left side 26. The amount of medial recess 38 is given by a negative value if the arch shape 44 has a recess, meanwhile by a positive value if the arch shape 44 has a protrusion, rather than the recess. Now, a foot width direction line 35 is defined as an imaginary line that passes through the deepest point 33 and orthogonal to the medial side reference line 36. An intersection of the contour line of the middle foot part 41 on the lateral side and the foot width direction line 35 is defined as a lateral side reference point 34. An imaginary line that passes through the lateral side reference point 34 and parallel to the right side 27 is defined as a lateral side reference line 37.

A lateral protrusion 39 calculated by the protrusion amount calculation unit 68 corresponds to the distance between an imaginary line passing through the most protruded point and an imaginary line passing through a predetermined reference point, in the outline shape 45 near the little toe ball. That is, the lateral protrusion 39 is a distance between the right side 27 passing through the lateral end point 29 and the lateral side reference line 37 passing through the lateral side reference point 34. The lateral protrusion 39 is given by a positive value if the outline shape 45 has a protrusion, meanwhile by a negative value if the outline shape 45 has a recess, rather than the protrusion.

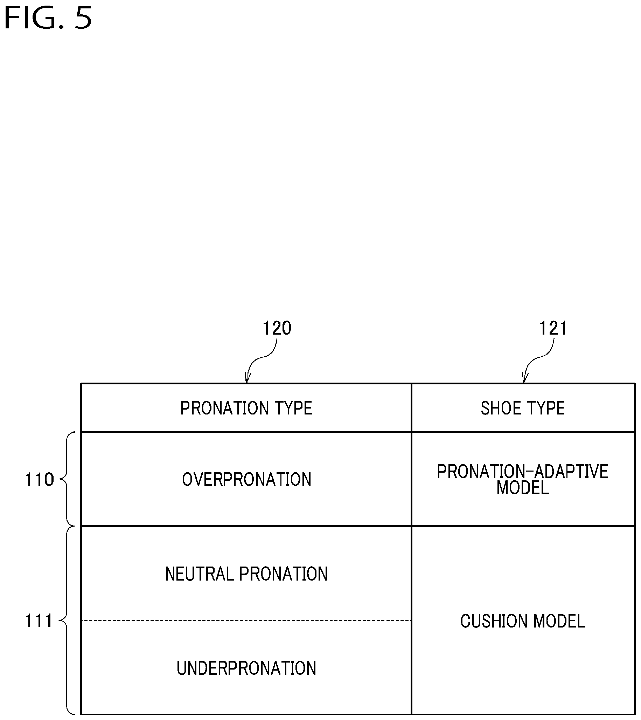

Fig. 5 summarizes relations between the pronation types and the shoe types. A pronation type 120 stored in the pronation type storage 84 includes
"neutral pronation" featured by moderate medial tilting of the heel upon landing, with a valgus angle within a threshold value X; "overpronation" featured by large medial tilting of the heel with a valgus angle exceeding the threshold value X; and "underpronation" featured by small medial tilting or lateral tilting of the heel. Note, the lateral tilting of the heel, alternatively referred to as "supination", is classified herein into "underpronation" featured by small medial tilting. With reference to the "neutral pronation", a case where the degree of pronation of the foot is larger than the "neutral pronation" is defined as a first type 110, and a case with an equal to or smaller degree is defined as a second type 111.

A shoe type 121 stored, as a shoemaking model, in the shoe type storage 86 includes a "pronation-adaptive model" that corresponds to the first type 110, and a "cushion model" that corresponds to the second type 111. The "pronation-adaptive model" is also referred to as "stability model". For a suppressive effect on the pronation, these models may have some hard piece arranged on the medial side of the heel, a mechanism for maintaining the arch, or a function of suppressing the medial twist of the middle foot part.

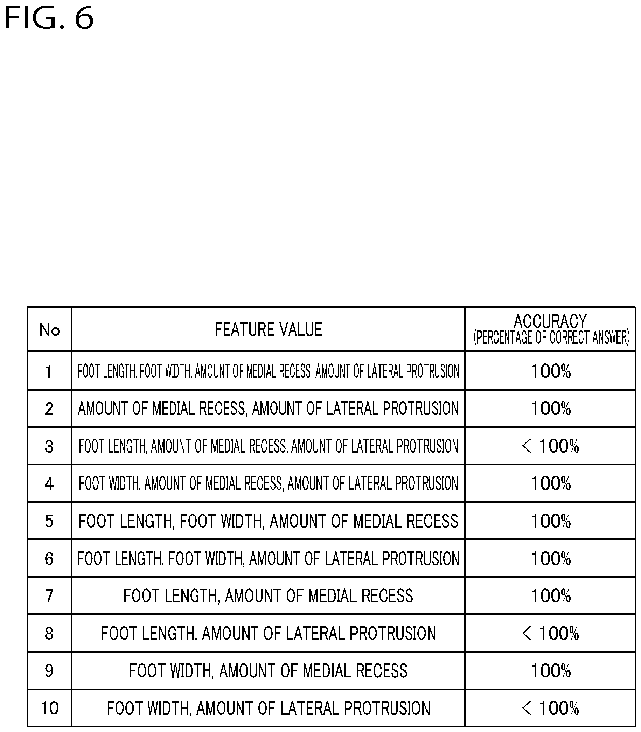

Fig. 6 summarizes results of collation between classification results of the subjects based on the decision tree analysis, and classification results of the pronation types based on the three-dimensional valgus angle. The first analysis was implemented by subjecting all of four factors including foot length, foot width, the amount of medial recess, and the amount of lateral protrusion, to the decision tree analysis. Results of the first analysis were found to coincide with the classification results of the pronation type based on the three-dimensional valgus angle, confirming that the pronation type was successfully estimated with 100% accuracy. The second analysis was implemented by subjecting two factors, which were the amount of medial recess and the amount of lateral protrusion, to the decision tree analysis. Also results of the second analysis were found to coincide with the classification results of the pronation type based on the three-dimensional valgus angle, confirming that the pronation type was successfully estimated with 100% accuracy.

The third analysis was implemented by subjecting three factors, which were foot length, the amount of medial recess, and the amount of lateral protrusion, to the decision tree analysis. Results of the third analysis were found partially not to coincide with the classification results of the pronation type based on the three-dimensional valgus angle, confirming an estimation accuracy of below 100%. The fourth analysis was implemented by subjecting three factors, which were foot width, the amount of medial recess, and the amount of lateral protrusion, to the decision tree analysis. Also results of the fourth analysis were found to coincide with the classification results of the pronation type based on the three-dimensional valgus angle, confirming that the pronation type was successfully estimated with 100% accuracy.

The fifth analysis was implemented by subjecting three factors, which were foot length, foot width, and the amount of medial recess, to the decision tree analysis. Also results of the fifth analysis were found to coincide with the classification results of the pronation type based on the three-dimensional valgus angle, confirming that the pronation type was successfully estimated with 100% accuracy. The sixth analysis was implemented by subjecting three factors, which were foot length, foot width, and the amount of lateral protrusion, to the decision tree analysis. Also results of the sixth analysis were found to coincide with the classification results of the pronation type based on the three-dimensional valgus angle, confirming that the pronation type was successfully estimated with 100% accuracy.

The seventh analysis was implemented by subjecting two factors, which were foot length and the amount of medial recess, to the decision tree analysis. Also results of the seventh analysis were found to coincide with the classification results of the pronation type based on the three-dimensional valgus angle, confirming that the pronation type was successfully estimated with 100% accuracy. The eighth analysis was implemented by subjecting two factors, which were foot length and the amount of lateral protrusion, to the decision tree analysis. Results of the eighth analysis were found partially not to coincide with the classification results of the pronation type based on the three-dimensional valgus angle, confirming an estimation accuracy of below 100%.

The ninth analysis was implemented by subjecting two factors, which were foot width and the amount of medial recess, to the decision tree analysis. Also results of the ninth analysis were found to coincide with the classification results of the pronation type based on the three-dimensional valgus angle, confirming that the pronation type was successfully estimated with 100% accuracy. The tenth analysis was implemented by subjecting two factors, which were foot width and the amount of lateral protrusion, to the decision tree analysis. Results of the tenth analysis were found partially not to coincide with the classification results of the pronation type based on the three-dimensional valgus angle, confirming an estimation accuracy of below 100%.

These experiments taught that the pronation type may be estimated with high accuracy, particularly when the decision tree analysis employed combinations of factors including the foot width and the amount of medial recess. In contrast, all of the decision tree analyses with the estimation accuracy fallen below 100% were found to employ combinations of the factors including the amount of lateral protrusion. Hence this embodiment will employ, as a preferred combination, a foot pronation estimation algorithm that includes at least the foot width and the amount of medial recess, which is exemplified by (1) three factors including foot length, foot width, and the amount of medial recess, or (2) three factors including foot width, the amount of medial recess, and the amount of lateral protrusion. Note, however, that modified examples may employ a foot pronation estimation algorithm based on a combination of two factors including foot width and the amount of medial recess, or based on a combination of three factors including foot length, foot width, and the amount of lateral protrusion, as a valid algorithm. Moreover, a foot pronation estimation algorithm based on all four elements of foot length, foot width, the amount of medial recess, and the amount of lateral protrusion is also valid.

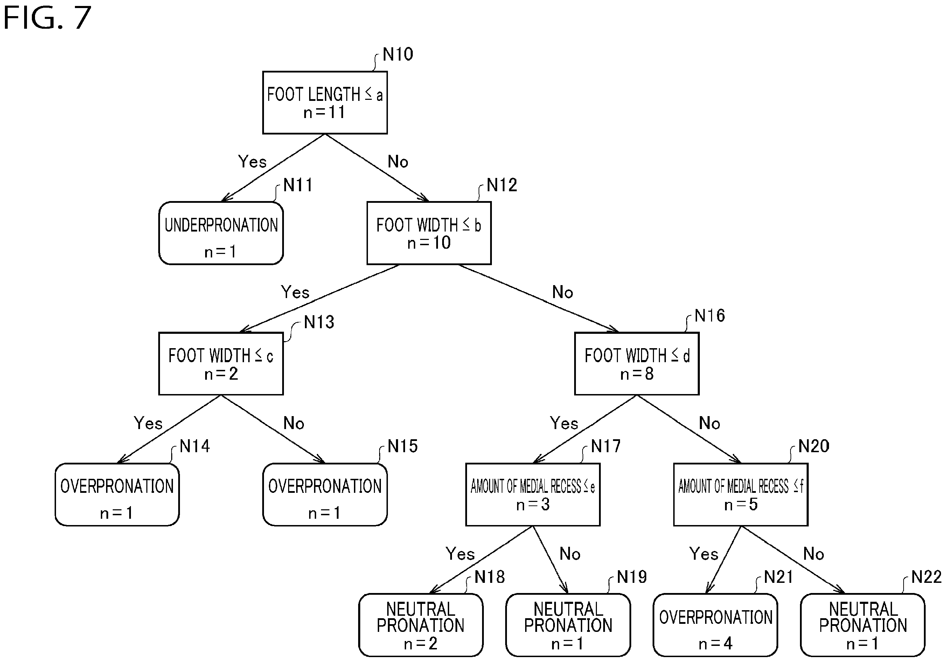

Fig. 7 is a tree diagram schematically illustrating results of the decision tree analysis of foot pronation estimation, based on three factors including foot length, foot width, and the amount of medial recess. In the example of Fig. 7, the measurement values with the number of samples of 11 (n = 11) are classified by the decision tree analysis, with a maximum depth of node set to five. Root node N10 has the "foot length" as the explanatory variable and a "predetermined value a" as the threshold value, both being determined in a regression manner, thus setting a branching condition stating whether or not the foot length is equal to or smaller than "a".

If the foot length is equal to or smaller than "a", leaf node N11 with a square error of 0 and the number of samples of one (n = 1) is classified into underpronation from the valgus angle. If the foot length exceeds "a", the branching is destined to node N12. Node N12 with the number of samples of ten (n = 10) has the "foot width" as the explanatory variable and a "predetermined value b" as the threshold value, both being determined in a regression manner, thus setting a branching condition stating whether or not the foot width is equal to or smaller than "b".

If the foot width is equal to or smaller than "b", the branching is destined to node N13. Node N13 with the number of samples of two (n = 2) has the "foot width" as an explanatory variable and a "predetermined value c" as a threshold value, both being determined in a regression manner, thus setting a branching condition stating whether or not the foot width is equal to or smaller than "c". If the foot width is equal to or smaller than "c", leaf node N14 with a square error of 0 and the number of samples of one (n = 1) will have a valgus angle exceeding the threshold value X, and is classified into overpronation. If the foot width exceeds "c", also leaf node N15 with a square error of 0 and the number of samples of one (n = 1) will have a valgus angle exceeding the threshold value X, and is classified into overpronation.

If the foot width exceeds "b" in node N12, the branching is destined to node N16. Node N16 with the number of samples of eight (n = 8) has the "foot width" as the explanatory variable and a "predetermined value d" as the threshold value, both being determined in a regression manner, thus setting a branching condition stating whether or not the foot width is equal to or smaller than "d". If the foot width is equal to or smaller than "d", the branching is destined to node N17. Node N17 with the number of samples of three (n = 3) has "the amount of medial recess" as the explanatory variable and a "predetermined value e" as the threshold value, both being determined in a regression manner, thus setting a branching condition stating whether or not the amount of medial recess is equal to or smaller than "e". If the amount of medial recess is equal to or smaller than "e", leaf node N18 with a square error of 0 and the number of samples of two (n = 2) is classified into neutral pronation from an averaged valgus angle. If the amount of medial recess exceeds "e", also leaf node N19 with a square error of 0 and the number of samples of one (n = 1) is classified into neutral pronation from the valgus angle.

If the foot width exceeds "d" in node N16, the branching is destined to node N20. Node N20 with the number of samples of five (n = 5) has the "the amount of medial recess" as the explanatory variable and a "predetermined value f" as the threshold value, both being determined in a regression manner, thus setting a branching condition stating whether or not the amount of medial recess is equal to or smaller than "f". If the amount of medial recess is equal to or smaller than "f", leaf node N21 with a square error of approximately 0.9 and the number of samples of four (n = 4) will have an averaged valgus angle exceeding the threshold value X, and is classified into overpronation. If the amount of medial recess exceeds "f", leaf node N22 with a square error of 0 and the number of samples of one (n = 1) is classified into neutral pronation from the valgus angle.

Classification by the decision tree analysis can determine the feature values suitable for the classification and the threshold values thereof in a regression manner, even if a sample having a largely deviated valgus angle, such as in leaf node N11, were contained. The classification may therefore take place appropriately, without affecting the accuracy of the overall estimation of foot pronation.

According to the procedures determined by the decision tree analysis of Fig. 7, a foot pronation estimation algorithm to be used by the pronation estimator 72 is determined. Now, the estimation process by the pronation estimator 72 only determines either the first type or the second type as summarized in Fig. 5, rather than determining all of overpronation, neutral pronation, and underpronation. An estimation process for distinguishing neutral pronation and underpronation is therefore unnecessary. Such estimation process is also unnecessary in a case where the branching is destined to the same classification, such as the branching from nodes N13 and N17. As a result, the foot pronation estimation algorithm to be employed by the pronation estimator 72 may be simplified as illustrated in the next drawing, which is simplified from the procedures based on the decision tree analysis illustrated in Fig. 7. Specific numerical values of the predetermined values a, b, d, and f that serve as the threshold values for the branching condition are determined by the decision tree analysis, which constitute the foot pronation estimation algorithm, and are stored in the reference storage 82 as the reference values for each feature value.

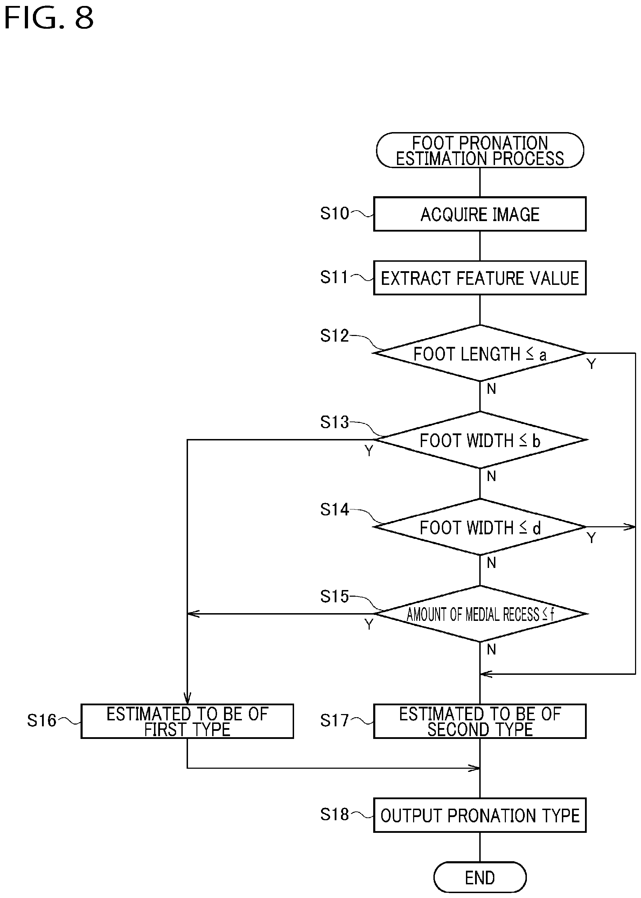

Fig. 8 is a flow chart illustrating a flow of a foot pronation estimation process with use of a foot pronation estimation algorithm based on three factors including foot length, foot width, and the amount of medial recess. The example illustrated in Fig. 8 employs the branching decisions that correspond to nodes N10, N12, N16, and N20 in the decision tree analysis illustrated in Fig. 7, as the foot pronation estimation algorithm. The image acquirer 54 acquires a top-view image of the foot of the measured person 10 (S10), and the feature value extractor 60 extracts the foot length, foot width, and the amount of medial recess as the feature values of the foot part (S11). If the foot length is equal to or smaller than the predetermined value a (Y in S12), the pronation estimator 72 estimates the pronation to be of the second type (S17). Meanwhile, if the foot length exceeds the predetermined value a (N in S12) and the foot width is equal to or smaller than the predetermined value b (Y in S13), the pronation is then estimated to be of the first type (S16). If the foot width exceeds the predetermined value b (N in S13), and the foot width is equal to or smaller than the predetermined value d (Y in S14), the pronation is then estimated to be of the second type (S17). If the foot width exceeds the predetermined value d (N in S14), the pronation is estimated to be of the first type (S16) if the amount of medial recess is equal to or smaller than the predetermined value f (Y in S15), meanwhile estimated to be of the second type (S17) if the amount of medial recess exceeds the predetermined value f (N in S15). The output unit 90 outputs the pronation type (S18) in response to the result of the estimation (S16, S17) as to whether the type applies to the first type or the second type, whereby the foot pronation estimation process comes to the end. In this flow, the processes in S12, S13, S14, and S15 are branch determination processes that respectively correspond to the nodes N10, N12, N16, and N20 in Fig. 7.

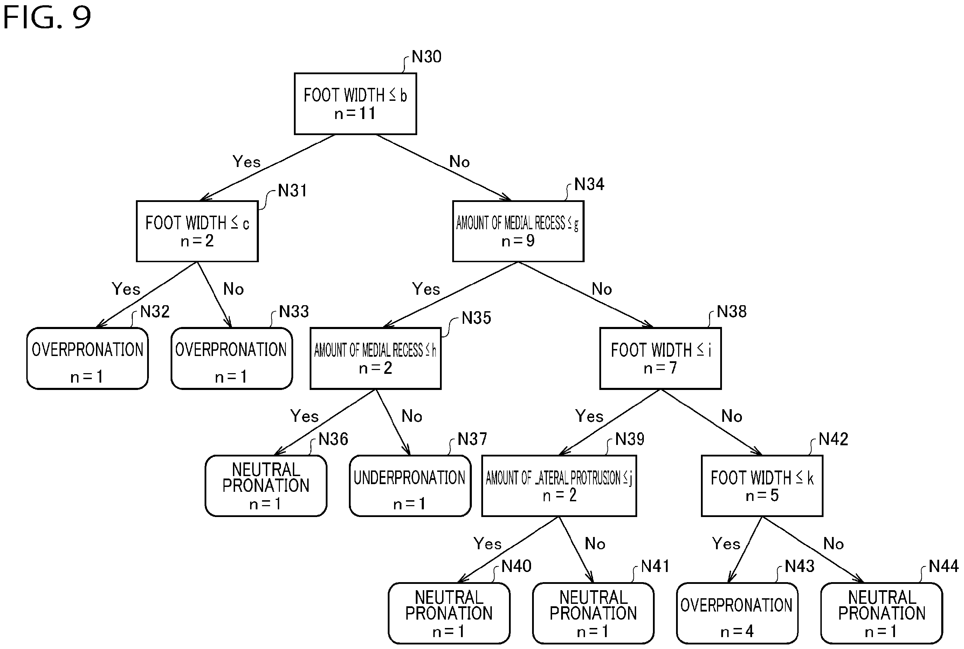

Fig. 9 is a tree diagram schematically illustrating results of the decision tree analysis of foot pronation estimation based on three factors including foot width, the amount of medial recess, and the amount of lateral protrusion. Also in the example of Fig. 9, the measurement values with the number of samples of 11 (n = 11) are classified by the decision tree analysis, with a maximum depth of node set to five. Root node N30 has the "foot width" as the explanatory variable and a "predetermined value b" as the threshold value, both being determined in a regression manner, thus setting a branching condition stating whether or not the foot width is equal to or smaller than "b".

If the foot width is equal to or smaller than "b", the branching is destined to node N31. Node N31 with the number of samples of two (n = 2) has the "foot width" as the explanatory variable and a "predetermined value c" as the threshold value, both being determined in a regression manner, thus setting a branching condition stating whether or not the foot width is equal to or smaller than "c". If the foot width is equal to or smaller than "c", leaf node N32 with a square error of 0 and the number of samples of one (n = 1) will have a valgus angle exceeding the threshold value X, and is classified into overpronation. If the foot width exceeds "c", also leaf node N33 with a square error of 0 and the number of samples of one (n = 1) will have a valgus angle exceeding the threshold value X, and is classified into overpronation.

If the foot width exceeds "b" in root node N30, the branching is destined to node N34. Node N34 with the number of samples of nine (n = 9) has "the amount of medial recess" as the explanatory variable and a "predetermined value g" as the threshold value, both being determined in a regression manner, thus setting a branching condition stating whether or not the amount of medial recess is equal to or smaller than "g".

If the amount of medial recess is equal to or smaller than "g", the branching is destined to node N35. Node N35 with the number of samples of two (n = 2) has "the amount of medial recess" as the explanatory variable and a "predetermined value h" as the threshold value, both being determined in a regression manner, thus setting a branching condition stating whether or not the amount of medial recess is equal to or smaller than "h". If the amount of medial recess is equal to or smaller than "h", leaf node N36 with a square error of 0 and the number of samples of one (n = 1) is classified into neutral pronation from the valgus angle. If the amount of medial recess exceeds "h", leaf node N37 with a square error of 0 and the number of samples of one (n = 1) is classified into underpronation from the valgus angle.

If the amount of medial recess exceeds "g" in node N34, the branching is destined to node N38. Node N38 with the number of samples of seven (n = 7) has the "foot width" as the explanatory variable and a "predetermined value i" as the threshold value, both being determined in a regression manner, thus setting a branching condition stating whether or not the foot width is equal to or smaller than "i". If the foot width is equal to or smaller than "i", the branching is destined to node N39. Node N39 with the number of samples of three (n = 3) has "the amount of lateral protrusion" as the explanatory variable and a "predetermined value j" as the threshold value, both being determined in a regression manner, thus setting a branching condition stating whether or not the amount of lateral protrusion is equal to or smaller than "j". If the amount of lateral protrusion is equal to or smaller than "j", leaf node N40 with a square error of 0 and the number of samples of one (n = 1) is classified into neutral pronation from the valgus angle. If the amount of lateral protrusion exceeds "j", also leaf node N41 with a square error of 0 and the number of samples of one (n = 1) is classified into neutral pronation from the valgus angle.

If the foot width exceeds "i" in node N38, the branching is destined to node N42. Node N42 with the number of samples of five (n = 5) has the "foot width" as the explanatory variable and a "predetermined value k" as the threshold value, both being determined in a regression manner, thus setting a branching condition stating whether or not the foot width is equal to or smaller than "k". If the foot width is equal to or smaller than "k", leaf node N43 with a square error of approximately 0.9 and the number of samples of four (n = 4) will have an averaged valgus angle exceeding the threshold value X, and is classified into overpronation. If the foot width exceeds "k", leaf node N44 with a square error of 0 and the number of samples of one (n = 1) is classified into neutral pronation from the valgus angle.

According to the procedures determined by the decision tree analysis of Fig. 9, a foot pronation estimation algorithm used by the pronation estimator 72 is determined. Now, the estimation process by the pronation estimator 72 only determines either the first type or the second type as summarized in Fig. 5, rather than determining all of overpronation, neutral pronation, and underpronation. An estimation process for distinguishing neutral pronation and underpronation is therefore unnecessary. Such estimation process is also unnecessary in a case where the branching is destined to the same classification, such as the branching from nodes N13 and N17. As a result, the foot pronation estimation algorithm to be employed by the pronation estimator 72 may be simplified as illustrated in the next drawing, which is simplified from the procedures based on the decision tree analysis illustrated in Fig. 9. In addition, node N39 where the branching is judged by the amount of lateral protrusion is destined to the neutral pronation irrespective of yes or no, and is therefore omitted in the foot pronation estimation algorithm illustrated in the next drawing. The amount of lateral protrusion is therefore not referred to in the foot pronation estimation process, thus making the process substantially equal to the foot pronation estimation process based on two elements including the foot width and the amount of medial recess. Specific numerical values of the predetermined values b, g, i and k that serve as the threshold values for the branching condition are determined by the decision tree analysis, which constitute the foot pronation estimation algorithm, and are stored in the reference storage 82 as the reference values for each feature value.

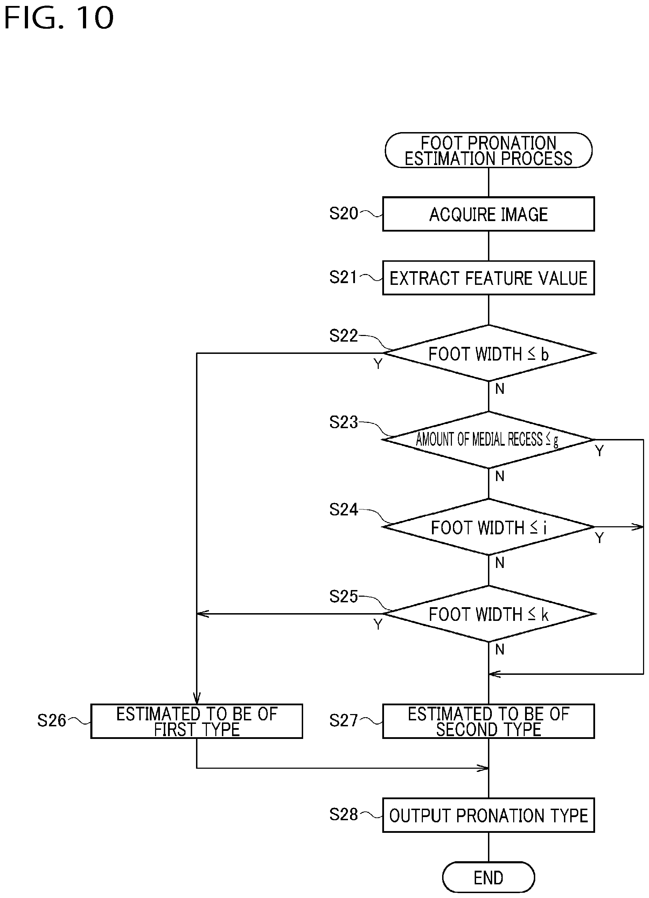

Fig. 10 is a flow chart illustrating a flow of the foot pronation estimation process with use of a foot pronation estimation algorithm based on three factors including foot width, the amount of medial recess, and the amount of lateral protrusion. The example illustrated in Fig. 10 employs the branching decisions that correspond to nodes N30, N34, N38, and N42 in the decision tree analysis illustrated in Fig. 9, as the foot pronation estimation algorithm. The image acquirer 54 acquires a top-view image of the foot of the measured person 10 (S20), and the feature value extractor 60 extracts the foot width, the amount of medial recess, and the amount of lateral protrusion as the feature values of the foot part (S21). In a modified example, the feature value extractor 60 may alternatively be designed to extract only the foot width and the amount of medial recess, as the feature values of the foot part. If the foot length is equal to or smaller than the predetermined value b (Y in S22), the pronation estimator 72 estimates the pronation to be of the first type (S26). Meanwhile, if the foot length exceeds the predetermined value b (N in S22) and the amount of medial recess is equal to or smaller than the predetermined value g (Y in S23), the pronation is then estimated to be of the second type (S27). If the amount of medial recess exceeds the predetermined value g (N in S23), and the foot width is equal to or smaller than the predetermined value i (Y in S24), the pronation is then estimated to be of the second type (S27). If the foot width exceeds the predetermined value i (N in S24), the pronation is estimated to be of the first type (S26) if the foot width is equal to or smaller than the predetermined value k (Y in S25), meanwhile estimated to be of the second type (S27) if the foot width exceeds the predetermined value k (N in S25). The output unit 90 outputs the pronation type (S28) in response to the result of the estimation (S26, S27) as to whether the type applies to the first type or the second type, whereby the foot pronation estimation process comes to the end. In this flow, the processes in S22, S23, S24, and S25 are branch determination processes that respectively correspond to the nodes N30, N34, N38, and N42 in Fig. 9.

The embodiments have been described above. The aforementioned embodiments are merely illustrative, so that those skilled in the art would easily understand that the individual constituents or combinations of various processes may be modified in various ways, and that also such modifications fall within the scope of the present disclosure.

In one modified example, also a side-view image of the foot may be supplementarily used besides the top-view image, from which a part of the feature values of a plurality of foot parts may be extracted. In another modified example, the pronation estimator 72 may acquire information regarding a preexisting condition of the measured person 10, and may supplementarily refer to the information regarding the preexisting condition in the pronation type estimation process.

Yet another modified example may control production percentages of the shoe product model corresponded to the first type and the shoe product model corresponded to the second type, with reference to a distribution of pronation types based on the estimation results of pronation types collected from a large number of measured persons 10. Yet another modified example may propose, to the measured person 10, a method of training the tibialis posterior muscle, trunk or buttocks, with reference to the estimation result of its pronation type. Yet another modified example not only may recommend the shoe product model according to the estimation result of the pronation type, but also may propose a design of an insole according to the estimation result of the pronation type.

The aforementioned embodiments will be generalized to give the modes below.
(1) A foot pronation estimation apparatus according to one mode of the present disclosure includes:
   an image acquirer structured to acquire at least a top-view image of a foot of a measured person, as an image that depicts a contour shape in a top view of the foot;
   a feature value extractor structured to extract, from the top-view image, a feature value of a predetermined part that depicts a top-view contour shape of the foot; and
   a pronation estimator structured to estimate a degree of pronation of the foot, with reference to comparison between the feature value of the predetermined part and a reference value.
   The foot pronation estimation apparatus according to (1) can provide a technique that enables the measured person to easily determine the pronation type.
(2) In the foot pronation estimation apparatus according to (1), the feature value extractor may be structured to extract, from the top-view image, feature values of a plurality of parts including at least a foot width and an amount of a recess of an arch shape, and
   the pronation estimator may be structured to estimate a degree of pronation of the foot, with reference to comparison of each of the feature values of the plurality of parts including at least the foot width and the amount of the recess of the arch shape, with an individually corresponded reference value.
(3) In the foot pronation estimation apparatus according to (2), the feature values of the plurality of parts may further include foot length.
(4) In the foot pronation estimation apparatus according to (2), the feature values of the plurality of parts may further include an amount of protrusion in an outline shape near a little toe ball.
(5) The foot pronation estimation apparatus according to any one of (1) to (4) may further include a storage structured to store a plurality of pronation types classified by the degree of pronation of foot, including at least a first type whose degree of pronation is larger than a predetermined standard, and a second type whose degree of pronation is equal to or smaller than the predetermined standard,
   wherein the pronation estimator may be structured to estimate which of the plurality of pronation types applies, with reference to comparison between the feature value of the predetermined part and the reference value.
(6) The foot pronation estimation apparatus according to any one of (1) to (5) may further include a storage structured to store a plurality of shoe types classified by the mode of dealing with pronation of foot, including at least a first shoe type adapted to a degree of pronation larger than a predetermined standard, and a second shoe type adapted to a degree of pronation equal to or smaller than the predetermined standard; and
   a type determiner structured to determine a shoe type recommendable for the measured person, from among the plurality of shoe types, with reference to the degree of pronation estimated by the pronation estimator.
(7) The foot pronation estimation apparatus according to any one of (1) to (6) may further include a storage structured to store a reference value related to the feature value of the plurality of parts,
   wherein the pronation estimator may be structured to estimate the degree of pronation, with reference to comparison of the extracted feature with the stored reference value.
(8) In the foot pronation estimation apparatus according to any one of (1) to (7), the image acquirer may be structured to acquire, as the top-view image, an image that depicts a top-view contour shape of the foot of the measured person in a sitting posture.
(9) In the foot pronation estimation apparatus according to any one of (1) to (8), the image acquirer may be structured to further acquire a side-view image of the foot of the measured person, and
   the feature value extractor may be structured to extract the feature value of the predetermined part, from at least either the top-view image or the side-view image.
(10) A method for estimating foot pronation according to one mode of the present disclosure may include:
   acquiring at least a top-view image of a foot of a measured person, as an image that depicts a contour shape in a top view of the foot;
   extracting, from the top-view image by image processing, a feature value of a predetermined part that depicts a top-view contour shape of the foot; and
   estimating a degree of pronation of the foot, with reference to a comparison process between the feature value of the predetermined part and a reference value.
(11) A foot pronation estimation program product according to one mode of the present disclosure may be structured to make a computer to implement:
   a function of acquiring at least a top-view image of a foot of a measured person, as an image that depicts a contour shape in a top view of the foot;
   a function of extracting, from the top-view image, a feature value of a predetermined part that depicts a top-view contour shape of the foot;
   a function of estimating a degree of pronation of the foot, with reference to comparison between the feature value of the predetermined part and a reference value; and
   a function of outputting information that represents a result of estimation.

## Claims

1. A foot pronation estimation apparatus (50) comprising:
an image acquirer (54) structured to acquire at least a top-view image of a foot of a subject, as an image that depicts a contour shape in a top view of the foot;
a feature value extractor (60) structured to extract a feature value of a predetermined part that depicts a top-view contour shape of the foot from the top-view image; and
a pronation estimator (72) structured to estimate a degree of pronation of the foot, with reference to comparison between the feature value of the predetermined part and a reference value.

2. The foot pronation estimation apparatus (50) according to claim 1, wherein the feature value extractor (60) is structured to extract the feature values of a plurality of parts including at least a foot width and an amount of a recess of an arch shape from the top-view image, and
the pronation estimator (72) is structured to estimate a degree of pronation of the foot, with reference to comparison of each of the feature values of the plurality of parts including at least the foot width and the amount of the recess of the arch shape, with an individually corresponded reference value.

3. The foot pronation estimation apparatus (50) according to claim 2, wherein the feature values of the plurality of parts further include foot length.

4. The foot pronation estimation apparatus (50) according to claim 2, wherein the feature values of the plurality of parts further include an amount of protrusion in an outline shape near a little to ball.

5. The foot pronation estimation apparatus (50) according to any one of claims 1 to 4, further comprising:
a pronation type storage (84) structured to store a plurality of pronation types classified by the degree of pronation of foot, including at least a first type whose degree of pronation is larger than a predetermined standard, and a second type whose degree of pronation is equal to or smaller than the predetermined standard,
wherein the pronation estimator (72) is structured to estimate which of the plurality of pronation types applies, with reference to comparison between the feature value of the predetermined part and the reference value.

6. The foot pronation estimation apparatus (50) according to any one of claims 1 to 5, further comprising:
a shoe type storage (86) structured to store a plurality of shoe types classified by the mode of dealing with pronation of foot, including at least a first shoe type adapted to a degree of pronation larger than a predetermined standard, and a second shoe type adapted to a degree of pronation equal to or smaller than the predetermined standard; and
a type determiner (74) structured to determine a shoe type recommendable for the subject, from among the plurality of shoe types, with reference to the degree of pronation estimated by the pronation estimator (72).

7. The foot pronation estimation apparatus (50) according to any one of claims 1 to 6, further comprising:
a reference storage (82) structured to store a reference value related to the feature value of the predetermined part,
wherein the pronation estimator (72) is structured to estimate the degree of pronation, with reference to comparison of the extracted feature with the stored reference value.

8. The foot pronation estimation apparatus (50) according to any one of claims 1 to 7, wherein the image acquirer (54) is structured to acquire, as the top-view image, an image that depicts a top-view contour shape of the foot of the subject in a sitting posture.

9. The foot pronation estimation apparatus (50) according to any one of claims 1 to 8, wherein the image acquirer (54) is structured to further acquire a side-view image of the foot of the subject, and
the feature value extractor (60) is structured to extract the feature value of the predetermined part, from at least either the top-view image or the side-view image.

10. A method for estimating foot pronation, the method comprising:
acquiring at least a top-view image of a foot of a subject, as an image that depicts a contour shape in a top view of the foot;
extracting a feature value of a predetermined part that depicts a top-view contour shape of the foot from the top-view image by image processing; and
estimating a degree of pronation of the foot, with reference to a comparison process between the feature value of the predetermined part and a reference value.

11. A non-transitory computer-readable storage medium storing a program for causing a computer to execute processing comprising:
acquiring at least a top-view image of a foot of a measured person, as an image that depicts a contour shape in a top view of the foot;
extracting a feature value of a predetermined part that depicts a top-view contour shape of the foot from the top-view image;
estimating a degree of pronation of the foot, with reference to comparison between the feature value of the predetermined part and a reference value; and
outputting information that represents a result of estimation.
